# EUROPEAN PATENT SPECIFICATION

(11) **EP 1 644 732 B1**
(45) Date of publication and mention of the grant of the patent: **24.02.2010**
(21) Application number: 04776817.1
(22) Date of filing: 18.06.2004
(51) Int. Cl.: G01N 33/50, A61K 49/00, A01K 67/027, C12Q 1/68, G01N 33/574

(54) **ANIMAL MODEL FOR THE ANALYSIS OF TUMOR METASTASIS**
TIERMODELL ZUR ANALYSE VON TUMORMETASTASIERUNG
MODELE ANIMAL UTILE POUR L'ANALYSE DES METASTASES TUMORALES

(30) Priority: 10.07.2003 US 487044 P
(43) Date of publication of application: 12.04.2006
(73) Proprietor: CENTRAL INSTITUTE FOR EXPERIMENTAL ANIMALS, Kawasaki-shi, Kanagawa 216-0001 (JP); Center for the Advancement of Health and Biosciences, Menlo Park, CA 94025 (US)
(72) Inventor: NAKAMURA, Masato, Setagaya-ku,Tokyo (JP); OHNISHI, Yasuyuki, Yokohama, Kanagawa (JP)
(74) Representative: Forrest, Graham Robert
(86) International application number: PCT/US2004/019697
(87) International publication number: WO 2005/013682

(56) References cited:
- WO-A-02/43477
- DEWAN M ZAHIDUNNABI ET AL: "Rapid tumor formation of human T-cell leukemia virus type 1-infected cell lines in novel NOD-SCID/gammacnull mice: Suppression by an inhibitor against NF-kappaB." JOURNAL OF VIROLOGY, vol. 77, no. 9, May 2003 (2003-05), pages 5286-5294, XP002309127 ISSN: 0022-538X
- TARBE N ET AL: "IDENTIFICATION OF RAT PANCREATIC CARCINOMA GENES ASSOCIATED WITH LYMPHOGENOUS METASTASIS" ANTICANCER RESEARCH, HELENIC ANTICANCER INSTITUTE, ATHENS,, GR, vol. 22, no. 4, July 2002 (2002-07), pages 2015-2028, XP009039609 ISSN: 0250-7005
- ANONYMOUS: "Atlas TM Glass Human 1.0 Microarray / State-of-the-art Atlas TM technology - now available on glass" CLONTECHNIQUES, [Online] April 2000 (2000-04), XP002324248 Retrieved from the Internet: URL:http://www.clontech.com/clontech/archi ve/APR00UPD/pdf/atlasglass.pdf> [retrieved on 2005-04-12]
- LACOBUZIO-DONAHUE C A ET AL: "DISCOVERY OF NOVEL TUMOR MARKERS OF PANCREATIC CANCER USING GLOBAL GENE EXPRESSION TECHNOLOGY" AMERICAN JOURNAL OF PATHOLOGY, PHILADELPHIA, PA, US, vol. 160, no. 4, April 2002 (2002-04), pages 1239-1249, XP008003747 ISSN: 0002-9440
- CRNOGORAC-JURCEVIC TATJANA ET AL: "Expression profiling of microdissected pancreatic adenocarcinomas" ONCOGENE, BASINGSTOKE, HANTS, GB, vol. 21, no. 29, 4 July 2002 (2002-07-04), pages 4587-4594, XP002267283 ISSN: 0950-9232
- HAN HAIYONG ET AL: "Identification of differentially expressed genes in pancreatic cancer cells using cDNA microarray" CANCER RESEARCH, AMERICAN ASSOCIATION FOR CANCER RESEARCH, BALTIMORE, MD, US, vol. 62, no. 10, 15 May 2002 (2002-05-15), pages 2890-2896, XP002267297 ISSN: 0008-5472
- SUN FANG-XIAN ET AL: "Efficacy of camptothecin analog DX-8951f (exatecan mesylate) on human pancreatic cancer in an orthotopic metastatic model." CANCER RESEARCH, vol. 63, no. 1, 1 January 2003 (2003-01-01), pages 80-85, XP002309311 ISSN: 0008-5472
- PROX DANIELA ET AL: "Treatment of human pancreatic cancer in mice with angiogenic inhibitors." WORLD JOURNAL OF SURGERY. APR 2003, vol. 27, no. 4, April 2003 (2003-04), pages 405-411, XP008040044 ISSN: 0364-2313
- KELLEY JOSEPH R ET AL: "CaSm antisense gene therapy: a novel approach for the treatment of pancreatic cancer." ANTICANCER RESEARCH. 2003 MAY-JUN, vol. 23, no. 3A, May 2003 (2003-05), pages 2007-2013, XP008040051 ISSN: 0250-7005
- ITO M ET AL: "NOD/SCID/GAMMAcnull mouse: an excellent recipient mouse model for engraftment of human cells" BLOOD, W.B.SAUNDERS COMPANY, ORLANDO, FL, US, vol. 100, no. 9, November 2002 (2002-11), pages 3175-3182, XP002981771 ISSN: 0006-4971 cited in the application
- DATABASE BIOSIS [Online] BIOSCIENCES INFORMATION SERVICE, PHILADELPHIA, PA, US; 16 November 2002 (2002-11-16), MIYAKAWA YOSHI ET AL: "Establishment of Human Multiple Myeloma Model Using Newly Developed Immunodeficinent NOD/SCID/IL-2Rgamma-/- (NOG) Mice." XP002309128 Database accession no. PREV200300337064 & BLOOD, vol. 100, no. 11, 16 November 2002 (2002-11-16), page Abstract No. 2366, 44TH ANNUAL MEETING OF THE AMERICAN SOCIETY OF HEMATOLOGY; PHILADELPHIA, PA, USA; DECEMBER 06-10, 2002 ISSN: 0006-4971
- HOTZ HUBERT G ET AL: "Animal models of exocrine pancreatic cancer" INTERNATIONAL JOURNAL OF COLORECTAL DISEASE, vol. 15, no. 3, June 2000 (2000-06), pages 136-143, XP002324710 ISSN: 0179-1958
- GUO QINGBIN M: "DNA microarray and cancer." CURRENT OPINION IN ONCOLOGY. JAN 2003, vol. 15, no. 1, January 2003 (2003-01), pages 36-43, XP008045590 ISSN: 1040-8746
- MON-NAI MAKOTO ET AL: "New model for hepatic metastasis of human pancreatic carcinoma using NOD/SCID/gammacnull (NOG) mice." PROCEEDINGS OF THE AMERICAN ASSOCIATION FOR CANCER RESEARCH ANNUAL MEETING, vol. 44, July 2003 (2003-07), page 582, XP001182951 & 94TH ANNUAL MEETING OF THE AMERICAN ASSOCIATION FOR CANCER RESEARCH; WASHINGTON, DC, USA; JULY 11-14, 2003 ISSN: 0197-016X
- MISSIAGLIA E ET AL: "ANALYSIS OF GENE EXPRESSION IN CANCER CELL LINES IDENTIFIES CANDIDATE MARKERS FOR PANCREATIC TUMORIGENESIS AND METASTASIS" INTERNATIONAL JOURNAL OF CANCER, NEW YORK, NY, US, vol. 112, no. 1, 2 June 2004 (2004-06-02), pages 100-112, XP008043556 ISSN: 0020-7136
- DATABASE BIOSIS [Online] BIOSCIENCES INFORMATION SERVICE, PHILADELPHIA, PA, US 1989 BAXTER A.G. ET AL: 'Comparison of high and low diabetes-incidence NOD mouse strains' Database accession no. PREV19899131121
- YAN G. ET AL: 'Reduced expression of Tap1 and Lmp2 antigen-processing genes in the nonobese diabetic (NOD) mouse due to a mutation in their shared bidirectional promoter' J. IMMUNOL. vol. 159, 1997, pages 3068 - 3080
- GAVIN A.L. ET AL: 'Extracellular mutations of non-obese diabetic mouse Fc gamma R1 modify surface expression and ligand binding' J. BIOL. CHEM. vol. 271, no. 29, 1996, pages 17091 - 17099

## Description

### Background of the Invention

### Field of the Invention

The present invention concerns the use of a transgenic animal model in a method for the analysis of tumor metastasis. In particular, the present invention provides methods for the study of pancreatic tumors or tumor cell lines in a transgenic (including knock out) mouse model.

### Related Art

Immunodeficient mice, such as athymic nude mice, C.B-17/severe combined immunodeficiency (*scid*) mice and NOD/SCID mice have been widely used as animal models in cancer metastasis research (Bruns et al., Int. J. Cancer 10:102(2):101-8 (2002); Ohta et al., Jpn. J. Cancer Chemother. 23:1669-72 (1996); Jimenez et al., Ann. Surg. 231:644-54 (2000)). Thus, such mouse models have been used for preclinical testing of new cancer drugs and for the detection of metastasis related genes (Bruns *et al., supra;* Ohta *et al., supra;* Jimenez *et al. supra;* Hotz et al., Pancreas 26:E89-98 (2003); Tarbe et al., Anticancer Res. 21:3221-8 (2001)). However, the use of these models for studying the metastases of human cancer cells has so far been limited, primarily due to the low efficiency of the incidence of cancer metastasis in the recipient mice, and the large cell number required to achieve the desired results. Hotz et al (International Journal of Colorectal Disease, vol. 15, no 3, 2000) reviews animal models of human pancreatic cancer.

Recently, to establish a more efficient animal recipient for xenotransplantation, a novel immunodeficiency mouse, NOD/SCID/γ_{c}^{null} (also referred to as NOD/ShiJic-scid with γ_{c}^{null}, or NOG) has been developed. NOG transgenic mice have been described as an excellent recipient mouse model for engraftment of human cells (Ito et al., Blood 100:3175-82 (2002)), and for the study of the *in vivo* development of human T cells from CD34(+) cells (Saito et al., Int. Immunol. 14:1113-24 (2002)). When human cord blood stem cells (CBSC) were preserved in NOG mice, CBSC were differentiated to T lymphocytes and migrated to the peripheral lymphoid organs (Yahata et al., J. Immunol. 169:204-9 (2002)).

Metastasis, including hepatic metastasis, is often observed in human cancer, including pancreatic cancer even in early stage, cancers of the digestive tract, including colorectal cancer and gastrointestinal cancer, lung cancer, and the like, and is one of the most frequent causes of cancer deaths. New strategies are necessary to manage cancer metastases, which, in turn, require the availability of appropriate and efficient animal models. Accordingly, there is a great unmet need for reliable animal models that enable the study of metastasis.

### Summary of the Invention

In one aspect, the present invention concerns a method for testing tumor metastasis, comprising monitoring the development of tumor metastasis in a mouse comprising a NOD/SCID/IL-2 receptor γ-null (_{γc}^{null}) mutation, wherein said mouse has been inoculated with a tumor cell from a metastatic pancreatic tumor or pancreatic tumor cell line.

In one embodiment, the metastasis is hepatic, bone, brain or lung metastasis, in particular, hepatic metastasis.

In another embodiment, the tumor cell is from a metastatic tumor cell line, which can, for example, be a strongly, moderately or lightly metastatic tumor cell line.

Pancreatic cancer cell lines suitable for the present invention include, for example, MIAPaCa-2, AsPC-1, PANC-1, Capan-1, and BxPC-3.

Inoculation can be performed, for example, by portal vein injection.

In one embodiment, at least about 1x10² cells have been inoculated, without any other pretreatment including irradiation or cytokine-medication.

In another embodiment, at least about 1x10³ cells have been inoculated.

In yet another embodiment, at least about 1x10⁴ cells have been inoculated.

The development of tumor metastasis can be monitored by methods known in the art, such as by observing the appearance and number of the metastatic nodules formed.

In another aspect, the invention concerns a method for testing a candidate anti-metastasis compound, comprising monitoring the effect of said candidate compound on a tumor metastasis, wherein said compound has been administered to a mouse comprising a NOD/SCID/_{γc}^{null} mutation and wherein said mouse has developed tumor metastasis as a result of inoculation with a tumor cell from a metastatic pancreatic tumor or pancreatic tumor cell line.

The test compound can be any kind of molecule, including, without limitation, a peptide, polypeptide, antibody or a non-peptide small molecule.

### Brief Description of the Drawings

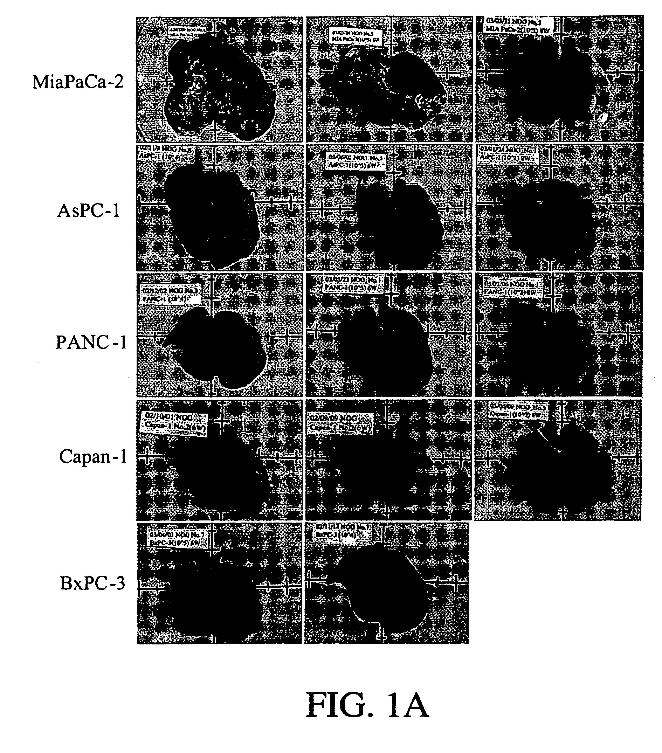

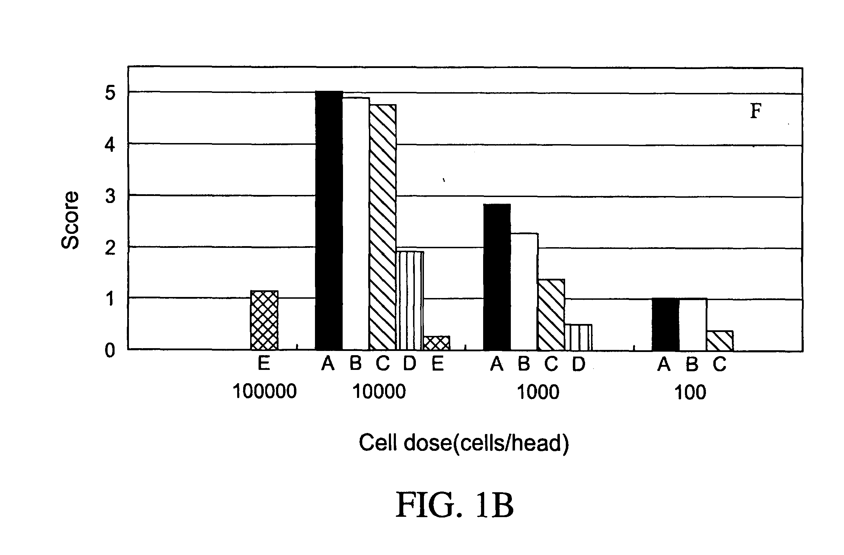

Figures 1A and B illustrate the incidences of hepatic metastasis and the number of liver foci in NOG mice following the inoculation of 1x10⁴, 1x10³ and 1x10² cells of the indicated pancreatic adenocarcinoma cells lines (MIAPaCa-2, AsPC-1, PANC-1, Capan-1, and BxPC-3.

### Detailed Description of the Preferred Embodiment

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, pancreatic cancer, prostate cancer, breast cancer, colorectal cancer, gastrointestinal cancer, colon cancer, lung cancer, hepatocellular cancer, cervical cancer, ovarian cancer, liver cancer, bladder cancer, cancer of the urinary tract, thyroid cancer, renal cancer, carcinoma, melanoma, and brain cancer.

The term "metastasis" is used herein in the broadest sense and refers to the spread of tumor, e.g. cancer from one part of the body to another. Tumors formed from cells that have spread are called secondary tumors, and contain the same type of cells as the original (primary) tumor. Thus prostate cancer that has metastasized to liver or bone is not liver or bone cancer, rather metastasized prostate cancer, as it still contains prostate cancer cells, regardless of their location.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

The terms "differentially expressed gene," "differential gene expression" and their synonyms, which are used interchangeably, refer to a gene whose expression is at a higher or lower level in one cell or cell type relative to another, or one patient or test subject relative to another. Thus, for example, differential gene expression can occur in normal cell/tissue/patient relative to a corresponding diseased cell/tissue/patient, or can reflect differences is gene expression pattern between different cell types or cells in different stages of development. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may, for example, be evidenced by a change in mRNA levels, surface expression, or secretion or other partitioning of a polypeptide. Differential gene expression may include a comparison of expression between two or more genes or their gene products, or a comparison of the ratios of the expression between two or more genes or their gene products, or a comparison of two differently processed products of the same gene. For the purpose of the present invention, "differential gene expression" is considered to be present when there is at least an about 2-fold, preferably at least about 2.5-fold, more preferably at least about 4-fold, even more preferably at least about 6-fold, most preferably at least about 10-fold difference between the expression of a given gene or gene product between the samples compared.

The term "microarray" refers to an ordered arrangement of hybridizable array elements on a substrate. The term specifically includes polynucleotide microarrays, such as cDNA and oligonucleotide microarrays, and protein arrays. In a particular embodiment, a microarray is an array of thousands of individual gene (DNA) sequences immobilized in a known order on a solid support. RNAs from different tissues are hybridized to the DNA on the chips. An RNA molecule will only bind to the DNA from which it was expressed. As a result, the relative expression of thousands of genes in biological samples (e.g. normal and diseased tissue, tissue treated or untreated with a certain drug, etc.) can be compared in a single assay. In a similar protein sequences can be displayed on a microarray chip and used to study protein-protein interactions, or differences in protein levels in different biological samples, e.g. tissues.

The term "polynucleotide," generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein includes triple-stranded regions comprising RNA or DNA or both RNA and DNA. The strands in such regions may be from the same molecule or from different molecules. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs.

The terms "transgenic animal" and "transgenic mouse" as well we their grammatical equivalents, are used to refer to animals/mice deliberately produced to carry a gene from another animal. Transgenic animals specifically include transgenic rodents, such as, for example, mice, rats, guinea pigs, and the like.

The term "xenotransplantation" is used in the broadest sense and refers to the transfer of living cells, tissues or organs from one animal species into another, including humans.

### B. Detailed Description

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); "Transgenic Mouse: Methods and Protocols" (Methods in Molecular Biology, Clifton N.J., Vol. 209, M.H. Hofker et al., eds.).

The present invention provides the use of a sensitive and reliable transgenic animal model in a method for the study of metastasis of pancreatic tumors. In particular, the present invention provides the use of a reproducible mouse model in a method for testing hepatic metastasis, which involves mammalian (e.g. human) pancreatic cancer cells that have been introduced into NOG mice.

NOG mice were developed at the Central Institute for Experimental Animals (CIEA, Kawasaki, Japan), and are also described in co-pending U.S. application Serial No. 10/221,549 filed on October 25, 2001.

In brief, to establish an improved animal recipient for xenotransplantation, NOD/SCID/_{γc}^{null} (NOG) mice double homozygous for the severe combined immunodeficiency (SCID) mutation and interleukin-2Rγ (IL-2Rγ) allelic mutation (γ_{c}^{null}) were generated by 8 backcross matings of C57BL/6J-γ_{c}^{null} mice and NOD/Shi-scid mice. When human CD34+ cells from umbilical cord blood were transplanted into this strain, the engraftment rate in the peripheral circulation, spleen, and bone marrow were significantly higher than that in NOD/Shiscid mice treated with anti-asialo GM1 antibody or in the β2-microglobulin-deficient. NOD/LtSz-scid (NOD/SCID/β₂ₘ^{null}) mice, which were as completely defective in NK cell activity as NOD/SCID/γ_{c}^{null} mice. The same high engraftment rate of human mature cells was observed in ascites when peripheral blood mononuclear cells were intraperitoneally transferred. In addition to the high engraftment rate, multilineage cell differentiation was also observed. Further, even 1 x 10(2) CD34+ cells could grow and differentiate in this strain. Based on these results, the NOD/SCID/γ_{c}^{null} mice were described to be superior animal recipients for xenotransplantation, especially for human stem cell assays. For further details see, e.g. Hiramatsu et al., Blood 100:3175-82 (2002).

It has now been found that the NOG mice are a superior mouse model for the study of human cancer metastasis. As such, this model can be used, for example, to screen and evaluate anti-cancer drugs and anti-metastasis drug candidates, and for the detection/screening of genes related to cancer metastasis, which, in turn, find utility in the diagnosis and/or treatment of metastatic cancer, and related conditions, including gene therapy treatment of metastatic cancer.

The mouse model for use in the method of the present invention is suitable for modeling and studying any kind of metastasis, including hepatic, bone, brain, and lung metastasis. Metastasis occur in all types

Methods of xenotransplantation are well known in the art and are illustrated in Example 1 below. Typically, cancer cells are transplanted into mice via tail vein injection, with or without prior immune-suppression, such as a sublethal dose of whole body irradiation and/or the administration of an immunosuppressant. For study of hepatic metastases, the cancer cells may be introduced into the animals by intrasplenic (portal vein) injection using an appropriate indwelling catheter. Pulmonary metastasis can be established, for example, by intravenous injection of tumor cells into the recipient animals, for example as described in Worth and Kleincrman; Clin Exp. Metastasis 17:501-6 (1999). The tumor cells may originate from tumor (cancer) cell lines, and from primary tumors (e.g. cancer) obtained from human or non-human subjects.

To study bone metastasis, macroscopic fragments of human fetal bone or mouse bone, may be implanted into NOG mice. A few weeks later, human tumor (cancer) cell lines or cells of primary tumors (cancer) can be injected either intravenously (colonization assay), or directly into the implanted tissue fragments. Tumor metastasis can be monitored by methods known in the art, including various imaging techniques and histologic examination.

When used for drug screening, following the engraftment of xenogenic tumor cells (either from cell lines or from primary tumors), the NOG mice that have developed metastatic cancer can be treated with the test compound(s), and any change in the number, size or other properties of the metastatic nodules as a result of drug treatment, and the viability of the test animals are monitored relative to untreated and/or positive control, where the positive control typically is an animal treated with a known anti-metastatic compound. The administration of the test compounds can be performed by any suitable route, including, for example, oral, transdermal, intravenous, infusion, intramuscular, etc. administration. Results obtained in this model can then be validated by follow-up pharmacokinetic, toxicologic, biochemical and immunologic studies, and ultimately human clinical studies.

Further details of the invention are illustrated by the following non-limiting example.

### Example 1

### Study of hepatic metastasis of human pancreatic cancer

### Materials and Methods

Male NOG mice and NOD/shiJic-scid mice of 7-9 weeks, which had been obtained from the Central Institute for Experimental Animals (CIEA, Kawasaki, Japan), were used in this study. The animals were kept under specific pathogen-free conditions according to the Guideline for the Regulation of Animal Experimentation of CIEA. All human pancreatic cancer cell lines used in this study were obtained from the American Type Culture Collection (Rockville, MD, USA). Culture media for AsPC-1 and Capan-1 were Dulbecco's modified Eagle's medium (DMEM) supplemented with 20% and 15% fetal bovine serum (FBS, Hyclone), respectively. MIAPaCa-2 and PANC-1 were maintained a culture of DMEM supplemented with 10% FBS. BxPC-3, Capan-2 and PL45 were maintained a culture of RPMI1640 (SIGMA, Cat.No.D6046 or D5796) supplemented with 10% FBS. These were maintained at 37°C in humidified atmosphere with 5% CO2. Experimental liver metastases were generated by intrasplenic/portal injection of cancer cells, as described previously (Khatib et al., Cancer Res. 62:242-50 (2002)). The animals were sacrificed 6-8 weeks later and liver metastases were enumerated immediately, without prior fixation. The metastatic lesions were evaluated on the following scale: O = No metastatic lesion; 1=1-10 metastatic lesions; 2 = 11 - 20 metastatic lesions; 3 = 21 or more metastatic lesions.

### Results

The incidences of hepatic metastases and the number of liver foci in NOG mice were far higher than those in NOD/SCID mice (Table 1 & Figures 1A and B). When the mice were inoculated with 1x10⁴ cells and sacrificed 6 weeks later, the incidences of hepatic metastases in NOG mice were as follows:
MIAPaCa-2, AsPC-1 and PANC-1 100%;
Capan-1 90%,
BxPC-3 12.5%; and
PL45 and Capan-2 0%.

In addition, metastases were apparent in 50-80% of NOG mice when 1x10³ MIAPaCa-2, AsPC-1, PANC-1 and Capan-1 cells were inoculated, and even when 1 × 10² MIAPaCa-2, AsPC-1 and PANC-1 cancer cells were inoculated, 37.5-71.4% of NOG mice show hepatic metastasis. These data indicate that the hepatic metastatic lesions in NOG mice inoculated with human pancreatic cancer cell lines were reproducibly formed in a dose dependent manner.

Typical macroscopic views of liver metastases in NOG mice and in NOD/SCID mice are shown in Figure 1A. The NOG mice injected with MLkPaCa-2, AsPC-1, PANC-1, Capan-1 and BxPC-3 cells showed multiple round metastases in the liver. However, the numbers of foci in these cell lines were wildly different depending on each cell line. Five out of 7 pancreatic cancer cell lines showed the metastatic potentials in NOG mouse, in contrast, no NOD/SCID mice showed hepatic metastasis under similar conditions, except for AsPC-1. As shown in Figures 1A and B, AsPC-1 showed the metastatic potentials in both mice lines, however, the degree of metastases in NOG mice were more severe than those in NOD/SCID mice.

Kusama et al. (Gastroenterology 122:308-17 (2002)) reported that metastatic lesions were apparent in 100% of athymic nude mice injected with 1x10⁶ AsPC-1 cells. These findings suggest AsPC-1 may be one of the cells with high metastatic potential, where the potential is dependent on the cell numbers injected.

The metastatic incidences of NOG mice inoculated with Capan-1 or BxPC-3 were faded away with decreasing the number of inoculating cells. In contrast, metastatic incidences were apparent in more than 50% of NOG mice inoculated with MIAPaCa-2 or AsPC-1 even when NOG mice were inoculated with only 1 × 10² cells (Table-1). These findings clearly indicate that NOG mice represent a highly superior metastasis model relative to other immunodeficient mouse models, and in particular NOD/SCID mice.

Most previous publications concerning hepatic metastases of human pancreatic cancer cells using nude mice report the intrasplenal inoculation of more than one million cancer cells (Shishido et al., Surg. Today 29(6):519-25 (1999); Nomura et al., Clin. Exp. Metastasis 19:391-9 (2002); and Ikeda et al., Jpn. J. Cancer Res. 81:987-93 (1990)). There are few reports of 100 % metastatic incidences, unless high metastatic clones derived from those cells lines were established. However, it is unlikely that more than 1 million cancer cells enter the liver at a stretch via the portal vein and form metastatic foci in pancreatic cancer patients, therefore, the current metastatic animal models are not representative of a typical human clinical situation.

In contrast, NOG mice represent an effective cancer metastasis model, which properly reflects the clinical conditions and behavior of human pancreatic cancer. Accordingly, the well-organized and reproducible hepatic metastases seen in NOG mice are useful in the study of hepatic metastasis of human pancreatic cancer and are expected to become the preferred model for screening and developing new anti-metastasis drugs.

It was reported that the murine NK activity were compensatory very high in immunodeficient animals such as nude, SCID and NOD/SCID mice, and contributed to the low rate of tumor growth and cancer metastasis (Shpitz et al., Anticancer Res. 14(5A):1927-34 (1994)). In contrast, Ito et al. (Blood 100:3221-8 (2001)) reported that NOG mice have no T, B and NK cells and decrease macrophage functions and dendritic cells functions. It is suggested that in the metastasis model using NOG mice, the metastatic potentials of cancer cells are detected without complex effects upon the immune system of the host, especially NK activity.

### Conclusions

The data presented demonstrate that the NOD/SCID/γ_{c}^{null} mouse model has a high potential to engraft xenogenic cells. Using this model for intrasplenic (portal vein) injection of cancer cells, reliable hepatic metastasis behavior of human pancreatic cells was observed. Four out of seven cell lines showed high hepatic metastatic potential (>80% incidence), and three of the cell lines studied showed low metastatic potential (<20% incidence) in NOG mice 6 weeks after transplantation only with 1 x 10⁴ cells. Moreover, hepatic metastases were apparent in NOG mice even when 1 x 10² cells of high metastatic cell lines were inoculated. Thus, the metastatic ability of cancer cells was demonstrated with a wide range of inoculated cell number, extending through 3 logarithmic orders of magnitude. The results also show that the NOG mouse model is clearly superior over the NOD/SCID model, which is currently considered the optimal animal model for study of cancer metastasis.

While the present invention is illustrated by way of certain embodiments, it is not so limited.

**Table 1**

| Cell line | Mice | Cell dose (cells/head) | Autopsy (week) | No. of mice with metastasis/total no. of mice (h) | Incidence (%) |
|---|---|---|---|---|---|
| MIA PaCa-2 | NOG | 1×10⁴ | 6 | 10/10 | 100.0 |
| pancreas; adenocarcinoma | | 1 ×10³ | 6 | 5/ 6 | 83.3 |
| | | 1×10² | 8 | 5/ 7 | 71.4 |
| | NOD/SCID | 1×10⁴ 6 | | 1/10 | 10.0 |
| | | 1×10³ | 6 | 0/ 7 | 0.0 |
| | | 1×10² | 8 | 0/ 6 | 0.0 |
| | | | | | |
| AsPC-1 | NOG | 1×10⁴ | 6 | 9/9 | 100.0 |
| pancreas; metastatic site: | | 1×10³ | 6 | 8/ 8 | 100.0 |
| ascites; adenocarcinoma | | 1×10² | 8 | 4/ 7 | 57.1 |
| | NOD/SCID | 1×10⁴ | 6 | 8/ 9 | 88.9 |
| | | 1×10³ | 6 | 1/ 8 | 12.5 |
| | | 1×10² | 8 | 0/ 6 | 0.0 |
| | | | | | |
| PANC-1 | NOG | 1×10⁴ | 6 | 8/ 8 | 100.0 |
| pancreas; adenocarcinoma | | 1×10³ | 6 | 6/ 8 | 75.0 |
| | | 1 ×10² | 8 | 3/ 8 | 37.5 |
| | NOD/SCID | 1×10⁴ | 6 | 0/10 | 0.0 |
| | | 1×10³ | 6 | 0/ 6 | 0.0 |
| | | 1×10² | 8 | 0/ 7 | 0.0 |
| | | | | | |
| Capan-1 | NOG | 1×10⁴ | 6 | 9/10 | 90.0 |
| pancreas; metastatic site: | | 1×10³ | 6 | 5/10 | 50.0 |
| liver; adenocarcinoma | | 1×10² | 8 | 0/ 8 | 0.0 |
| | NOD/SCID | 1×10⁴ | 6 | 0/10 | 0.0 |
| | | 1×10³ | 6 | 0/10 | 0.0 |
| | | 1×10² | 8 | 0/ 6 | 0.0 |
| | | | | | |
| BXPC-3 | NOG | 1×10⁵ | 6 | 8/ 8 | 100.0 |
| pancreas; adenocarcinoma | | 1×10⁴ | 6 | 1/ 8 | 12.5 |
| | NOD/SCID | 1×10⁵ | 6 | 0/ 8 | 0.0 |
| | | 1×10⁴ | 6 | 0/ 6 | 0.0 |
| Capan-2 | NOG | 1×10⁵ | 6 | 0/ 8 | 0.0 |
| pancreas; adenocarcinoma | | 1×10⁴ | 6 | 0/10 | 0.0 |
| | NOD/SCID | 1×10⁵ | 6 | 0/ 8 | 0.0 |
| | | | | | |
| PL45 | NOG | 1×10⁵ | 6 | 0/ 8 | 0.0 |
| Ductal adenocarcinoma; | | 1×10⁴ | 6 | 0/10 | 0.0 |
| pancreas | NOD/SCID | 1×10⁵ | 6 | 0/ 8 | 0.0 |

## Claims

1. A method for testing tumor metastasis, comprising monitoring the development of tumor metastasis in a mouse comprising a NOD/SCID/IL-2 receptor γ-null (_{γc}^{null}) mutation, wherein said mouse has been inoculated with a tumor cell from a metastatic pancreatic tumor or pancreatic tumor cell line.

2. The method of claim 1 wherein the tumor is cancer.

3. The method of claim 1 wherein the metastasis is selected from the group consisting of hepatic, bone, brain and lung metastases.

4. The method of claim 3 wherein the metastasis is hepatic metastasis.

5. The method of claim 4 wherein the tumor cell is from a metastatic tumor cell line.

6. The method of claim 5 wherein the tumor cell line is a strongly metastatic tumor cell line.

7. The method of claim 5 wherein the tumor cell line is selected from the group consisting of MIAPaCa-2, AsPC-1, PANC-1, Capan-1, and BxPC-3.

8. The method of claim 1 wherein said tumor cell has been inoculated into said mouse by portal vein injection.

9. The method of claim 8 wherein at least about 1×10² cells have been inoculated.

10. The method of claim 8 wherein at least about 1×10³ cells have been inoculated.

11. The method of claim 8 wherein at least about 1×10⁴ cells have been inoculated.

12. The method of claim 1 wherein the development of tumor metastasis is monitored by observing the appearance and number of the metastatic nodules formed.

13. A method for testing a candidate anti-metastasis compound, comprising monitoring the effect of said candidate compound on a tumor metastasis, wherein said compound has been administered to a mouse comprising a NOD/SCID/_{γc}^{null} mutation and wherein said mouse has developed tumor metastasis as a result of inoculation with a tumor cell from a metastatic pancreatic tumor or pancreatic tumor cell line.

14. The method of claim 13 wherein said metastasis is hepatic metastasis.

15. The method of claim 14 wherein said NOD/SCID/_{γc}^{null} mouse has developed hepatic metastasis as a result of inoculation with a metastatic cancer cell line.

16. The method of claim 15 wherein the cancer cell line is a metastatic pancreatic adenocarcinoma cell line.

17. The method of claim 16 wherein said metastatic pancreatic adenocarcinoma cell line is selected from the group consisting of MIAPaCa-2, AsPC-1, PANC-1, Capan-1, and BxPC-3.

18. The method of claim 13 wherein said test compound has been administered orally.

19. The method of claim 13 wherein said test compound has been administered intravenously.

20. The method of claim 13 wherein said test compound is selected from the group consisting of peptides, polypeptides, antibodies and non-peptide small molecules.

## Patentansprüche

1. Verfahren zum Testen einer Tumormetastase, umfassend das Überwachen der Entwicklung einer Tumormetastase in einer Maus, umfassend die γ-null- (_{γc}^{null}-) Mutation eines NOD/SCID/IL-2-Rezeptors, worin der Maus eine Tumorzelle von einem metastatischen Pankreastumor oder einer Pankreastumorzelllinie geimpft wurde.

2. Verfahren nach Anspruch 1, worin der Tumor Krebs ist.

3. Verfahren nach Anspruch 1, worin die Metastase aus der aus Leber-, Knochen-, Gehirn- und Lungenmetastasen bestehenden Gruppe ausgewählt ist.

4. Verfahren nach Anspruch 3, worin die Metastase eine Lebermetastase ist.

5. Verfahren nach Anspruch 4, worin die Tumorzelle von einer metastatischen Tumorzelllinie stammt.

6. Verfahren nach Anspruch 5, worin die Tumorzelllinie eine stark metastatische Tumorzelllinie ist.

7. Verfahren nach Anspruch 5, worin die Tumorzelllinie aus der aus MIAPaCa-2, AsPC-1, PANC-1, Capan-1 und BxPC-3 bestehenden Gruppe ausgewählt ist.

8. Verfahren nach Anspruch 1, worin die Tumorzelle der Maus mittels Injektion in die Pfortader geimpft wurde.

9. Verfahren nach Anspruch 8, worin zumindest etwa 1×10² Zellen geimpft wurden.

10. Verfahren nach Anspruch 8, worin zumindest etwa 1×10³ Zellen geimpft wurden.

11. Verfahren nach Anspruch 8, worin zumindest etwa 1×10⁴ Zellen geimpft wurden.

12. Verfahren nach Anspruch 1, worin die Entwicklung einer Tumormetastase durch die Beobachtung des Aussehens und der Anzahl der gebildeten metastatischen Knötchen überwacht wird.

13. Verfahren zum Testen einer Anti-Metastasen-Kandidatenverbindung, umfassend die Überwachung der Wirkung der Kandidatenverbindung auf eine Tumormetastase, worin die Verbindung einer Maus verabreicht wurde, die eine NOD/SCID/_{γc}^{null}-Mutation aufweist, und worin die Maus in Folge des Impfens einer Tumorzelle von einem metastatischen Pankreastumor oder einer Pankreastumorzelllinie eine Tumormetastase entwickelt hat.

14. Verfahren nach Anspruch 13, worin die Metastase eine Lebermetastase ist.

15. Verfahren nach Anspruch 14, worin die NOD/SCID/_{γc}^{null}-Maus in der Folge des Impfens einer metastatischen Krebszelllinie eine Lebermetastase entwickelt hat.

16. Verfahren nach Anspruch 15, worin die Krebszelllinie eine metastatische Pankreasadenokarzinomzelllinie ist.

17. Verfahren nach Anspruch 16, worin die metastatische Pankreasadenokarzinomzelllinie aus der aus MIAPaCa-2, AsPC-1, PANC-1, Capan-1 und BxPC-3 bestehenden Gruppe ausgewählt ist.

18. Verfahren nach Anspruch 13, worin die Testverbindung oral verabreicht wurde.

19. Verfahren nach Anspruch 13, worin die Testverbindung intravenös verabreicht wurde.

20. Verfahren nach Anspruch 13, worin die Testverbindung aus der aus Peptiden, Polypeptiden, Antikörpern und kleinen Molekülen, die keine Peptide sind, bestehenden Gruppe ausgewählt ist.

## Revendications

1. Procédé pour tester des métastases tumorales, consistant à surveiller le développement de métastases tumorales chez une souris comprenant une mutation NOD/SCID/nulle pour la chaîne y du récepteur de l'IL-2 (_{γc}^{null}), où ladite souris a subi une inoculation avec une cellule tumorale issue d'une tumeur pancréatique métastatique ou d'une lignée de cellules tumorales pancréatiques.

2. Procédé selon la revendication 1, dans lequel la tumeur est un cancer.

3. Procédé selon la revendication 1, dans lequel la métastase est sélectionnée dans le groupe consistant en des métastases hépatiques, osseuses, cérébrales et pulmonaires.

4. Procédé selon la revendication 3, dans lequel la métastase est une métastase hépatique.

5. Procédé selon la revendication 4, dans lequel la cellule tumorale est une lignée de cellules tumorales métastatiques.

6. Procédé selon la revendication 5, dans lequel la lignée de cellules tumorales est une lignée de cellules tumorales fortement métastatiques.

7. Procédé selon la revendication 5, dans lequel la lignée de cellules tumorales est sélectionnée dans le groupe consistant en MIAPaCa-2, AsPC-1, PANC-1, Capan-1, et BxPC-3.

8. Procédé selon la revendication 1, dans lequel ladite cellule tumorale a été inoculée chez ladite souris par une injection dans la veine porte.

9. Procédé selon la revendication 8, dans lequel au moins environ 1 x 10² cellules ont été inoculées.

10. Procédé selon la revendication 8, dans lequel au moins environ 1 x 10³ cellules ont été inoculées.

11. Procédé selon la revendication 8, dans lequel au moins environ 1 x 10⁴ cellules ont été inoculées.

12. Procédé selon la revendication 1, dans lequel le développement des métastases tumorales est surveillé en observant l'apparence et le nombre de nodules métastatiques formés.

13. Procédé pour tester un composé anti-métastatique candidat, consistant à surveiller l'effet dudit composé candidat sur une métastase tumorale, où ledit composé a été administré à une souris comprenant une mutation NOD/SCID/_{γc}^{null} et où ladite souris a développé une métastase tumorale suite à une inoculation avec une cellule tumorale issue d'une tumeur pancréatique métastatique ou d'une lignée de cellules tumorales pancréatiques.

14. Procédé selon la revendication 13, dans lequel ladite métastase est une métastase hépatique.

15. Procédé selon la revendication 14, dans lequel ladite souris NOD/SCID/_{γc}^{null} a développé une métastase hépatique suite à une inoculation avec une lignée de cellules cancéreuses métastatiques.

16. Procédé selon la revendication 15, dans lequel la lignée de cellules cancéreuses est une lignée de cellules d'adénocarcinome pancréatique métastatique.

17. Procédé selon la revendication 16, dans lequel ladite lignée de cellules d'adénocarcinome pancréatique métastatique est sélectionnée dans le groupe consistant en MIAPaCa-2, AsPC-1, PANC-1, Capan-1, et BxPC-3.

18. Procédé selon la revendication 13, dans lequel ledit composé de test a été administré par voie orale.

19. Procédé selon la revendication 13, dans lequel ledit composé de test a été administré par voie intraveineuse.

20. Procédé selon la revendication 13, dans lequel ledit composé de test est sélectionné dans le groupe consistant en des peptides, des polypeptides, des anticorps et des petites molécules non peptidiques.
